# EUROPEAN PATENT APPLICATION

(11) **EP 2 957 303 A1**
(43) Date of publication of application: **23.12.2015**
(21) Application number: 14173247.9
(22) Date of filing: 20.06.2014
(51) Int. Cl.: A61L 15/58, A61L 24/04, C08K 5/10, C09J 167/06, C08L 67/06

(54) **Curable composition and skin adhesive**

(71) Applicant: NITTO DENKO CORPORATION, Osaka 567 (JP)
(72) Inventor: Schüwer, Nicolas, 1010 Lausanne (CH)
(74) Representative: Hoffmann Eitle

(57) **Abstract**

The present invention pertains to compositions containing a polycondensate and optionally an oil component, wherein the polycondensate is derived from a dicarboxylic acid component, a diol component and an ethylenically unsaturated dicarboxylic acid component. The composition of the invention may be cross-linked to yield an adhesive composition. The composition of the invention and the adhesive composition of the invention may be used on substrates such as tapes and patches in the medical field.

## Description

### 1. Technical Field of the Invention

The present invention relates to a curable composition comprising: a polycondensate of a dicarboxylic acid, a diol, and a polymerizable ethylenic derivative; and optionally an oil additive. The invention is also directed to an adhesive composition as well as a method for preparing and/or manufacturing the adhesive composition, and to the use of the radiation-curable composition or adhesive composition for laminating a substrate such as a sheet, film, tape and/or patch.

### 2. State of the art

US 2012/0202058 A1 relates to polyester based pressure-sensitive adhesive sheet for surface protection. The polyester is composed of bio-based raw material such a lactic acid, a dibasic acid unit (e.g. dimer fatty dicarboxylic acid) and a glycol unit. The polyester is cross-linked using a polyfunctional isocyanyate.

EP 2 567 996 A1 and WO 2011/023255 A1 relate to bio-based pressure sensitive adhesive obtained by chemical crosslinking of a polycondensate of, for instance, dimer of a fatty alcohol and dimer of a fatty acid. As for US 2012/0202058 A1, the cross-linking technology reported in these patents may not be the most desired for biomedical applications since they contain chemicals presenting undesired biological effects.

WO 97/27253 A1 relates to a radiation-curable powder paint binder composition comprising a resin and an optional cross-linker in which more than 0.5 mol % of the total amount of polymerizable ethylenic unsaturation of the binder composition results from itaconic acid ester units.

US 3,989,609 relates to polyester based coatings in which itaconic acid can be used as a co-monomer that is reacted with, for instance, polyamide leading to a UV-curable pre-polymer. The formulation described in this patent can find use as radiation curable resistant coatings.

WO 2010/108965 A1 relates to unsaturated polyester resins comprising itaconate, citraconate and/or mesaconate ester units as reactive unsaturation. This polyester resin is claimed to be suitable for the manufacturing of structural part.

US 3,299,010 relates to pressure-sensitive adhesive polymers and tapes in which itaconic acid can be used as a co-monomer to form polymers having polyamide or polyacrylate backbones. The preparation of the adhesive sheets in accordance with this patent document is disadvantageous in that it requires substantial amounts of organic solvents, either in the initial polymerization step or in the cross-linking step if the initial polymerization reaction is a polycondensation.

US 5780151 A relates to radiation cross-linkable water-dispersible adhesive composition usable as a hot melt adhesive. The water-dispersible adhesive composition comprises a branched water-dispersible radiation cross-linkable polyester composition made of six components including a difunctional dicarboxylic acid, at least one difunctional sulfomonomer, at least one diol or a mixture of diol and diamine, a difunctional monomer reactant, a multifunctional reactant containing at least three functional groups; and an unsaturated mono- or dicarboxylic acid. Furthermore, polycondensation processes leading to the uncured and unsaturated polyesters are disclosed, which are also cured via UV-radiation. This adhesive formulation is meant to be used in forming paper and plastic articles and other products that can be recycled.

US 6,465,537 B1 relates to photo-curable composition which can be converted to a tackifying polymer via radical polymerization and finally caused to cure via cationic polymerization. The composition contains a compound having at least one free-radically polymerizable unsaturated bond in a molecule, a compound having at least one epoxy group in a molecule, a free-radical polymerization catalyst, a cationic polymerization catalyst and a cyclic compound.

US 2010/0006217 A1 relates to solvent-free, reactive composition containing an unsaturated polymer backbone. This patent describes reactive composition comprising at least a photoinitiator and a polymer (polyester, polyurethane and/or polyamide) containing an in-chain olefinically unsaturated group which is capable of reacting under UV-radiation.

EP 1 725 626 B1 relates to radiation-curable adhesive compositions comprising at least one tackifier resin and at least one radiation-curable composition; wherein the tackifier resin comprises monomer repeating units from at least one aromatic monomer and at least one acrylate monomer. This invention is claimed to have no significant skin sensitization properties.

WO 2013/025955 A1 relates to silicone-based radiation-cured adhesives containing a plurality of absorbent fibers dispersed within a hydrophilic adhesive. This adhesive composition can adhere to a variety of surfaces, such as skin, and can be easily remove from the surface.

### 3. Problem solved by the invention

Having regard to the state of the art summarized above, there remains a need for curable compositions and adhesives obtainable therefrom, as well as medical devices such as a tape or a patch, which simultaneously provide satisfactory adhesiveness and skin friendliness both during use and at the time of removing the device.

Furthermore, unlike most of the curable compositions reported so far, the invention described herein can be performed with building blocks that are derivable from natural and renewable resources. A further advantage is that the associated processes of the invention are solvent-free or require lower amounts of solvent than the prior art methods for preparing radiation-curable compositions, adhesives and/or medical devices comprising the radiation-curable compositions or adhesive. Therefore the carbon footprint and production costs compared to the prior art is lower.

In addition to the above benefits, the compositions and adhesives in accordance with the present invention exhibit a tuneable adhesion level and a high level of transparency. Hence, they are suitable for biomedical applications.

It is also the aim of the present invention to produce a skin adhesive that possesses equivalent or enhanced properties as compared to already known adhesives (silicon or acrylic based) but at advantageous production and raw material costs.

### 4. Summary of the Invention

The present invention solves the problems mentioned herein by providing a composition comprising: a polycondensate (A); and optionally an oil additive (B); wherein the polycondensate (A) contains repeating units derived from: a dicarboxylic acid (a); a diol (b); and an ethylenically unsaturated diacid (c); wherein the weight average molecular weight of the polycondensate (A) is from 35,000 g/mol to 95,000 g/mol; and, if present, the amount of the oil additive (B) in the composition is from 0.01 to 1.5 parts by weight, preferably 0.2 to 1.2 parts by weight, based on 1 part by weight of the polycondensate (A).

The present invention also provides an adhesive composition prepared from the compositions of the invention in which the polycondensate (A) has been at least partially cross-linked, as well as substrates such as medical tapes or patches that comprise the adhesive.

The present invention also provides a method for producing the adhesive composition as well as a method for producing medical devices such as tapes or patches that comprise the adhesive.

Preferred embodiments of the invention are defined in the dependent claims and throughout the description.

### 5. Detailed Description of the Invention

### 5.1 Definitions

The term ***"adhesive** gel **content value"*** refers to the value determined by the known method reported earlier (Vendamme R. and Eevers W. Macromolecules, 2013, 46 (9), 3395-3405).

The term ***"α transition**"* refers to the value determined by rheology. Rheological measurements were performed on a Discovery HR-2 hybrid rheometer (TA Instruments) in oscillation mode using parallel plate geometry. The α transition was measured by monitoring tan δ by scanning the temperature from -100°C to 150°C (ramp of 5 K/min) while imposing a constant strain of 0.4% and at a frequency of 1 Hz (linear regime conditions).

The term ***"peel force"*** or *"**adhesion level"*** refers to the force required to remove the tape once it is applied to human skin. It can be measured using a Shimadzu Ag-X universal tensile testing machine equipped with 1000N force cell.

Unless specified otherwise, references to the ***molecular** weight* of a polymeric substance are to be understood as references to the weight average molecular weight, which is determined by GPC using Shimadzu GPC system equipped with a differential refractive index detector and calibrated with polystyrene standards.

Unless specified otherwise, amount ***indications in*** % are to be understood as indications in weight %.

The *"**acid value"*** is defined as the mass of potassium hydroxide (in mg) required to neutralized 1g of polycondensate, and is measured by direct titration using a standard ethanolic potassium hydroxide solution.

The *"**hydroxyl value**"* is defined as the mass of potassium hydroxide (in mg) equivalent to the hydroxyl content of 1g of polycondensate. It is measured by acetylation of the polycondensate followed by hydrolysation of the excess of acetic anhydride. The acetic acid formed is subsequently titrated with an ethanolic potassium hydroxide solution.

The term *"**fatty acid**"* is meant to refer to carboxylic acids having a long aliphatic chain, which may be saturated or may contain one or more unsaturations. The aliphatic chain may range from 4 to 30 carbon atoms, preferably from 6 to 28 carbon atoms, more preferably from 8 to 25 carbon atoms and even more preferably from 10 to 22 carbon atoms.

The term *"**fatty alcohol**"* is meant to refer to an aliphatic alcohol having a long aliphatic chain, which may be saturated or may contain one or more unsaturations. The aliphatic chain may range from 4 to 30 carbon atoms, preferably from 6 to 28 carbon atoms, more preferably from 8 to 25 carbon atoms and even more preferably from 10 to 22 carbon atoms.

The term ***"bio-based"*** is meant to refer to substances derived from renewable starting materials (as opposed to mineral oil-derived starting materials) by isolation and optionally one or more chemical transformations.

*"**Suitability for topical administration to the human skin"*** means in the context of the present application that the substance of interest must be authorized by European regulatory bodies for incorporation into medical preparations that are to be administered to the human skin. The final adhesive composition must be suitable for topical administration to the human skin. This means that the cross-linked polycondensate, the optional oil additive and any other additional components must be suitable for administration to human skin. However, it does not mean that individual raw materials must be suitable for administration to human skin if these are consumed completely during the manufacturing process, or if residual raw materials remain at acceptably low levels or the product can be purified such that the purified product contains no or only acceptably low levels of potentially harmful raw materials.

In the context of the present application, the term *"**comprising**"* is meant to introduce an open list, such that further unmentioned members are not excluded. However, the use of "comprising" is intended to specifically characterize also the scenario, wherein no further unmentioned members are present. In other words, a disclosure of "comprising" is to be understood as a disclosure of "consisting of" as one distinct option.

In the context of the present invention, the reference to a feature being a ***"preferred"*** feature is meant to indicate that it is preferred to combine this feature with other preferred features of the invention. It is intended by the inventors, and should therefore be understood by the reader, that all such combinations of preferred features with other preferred features are directly and unambiguously disclosed. The following example is meant to serve as an illustration of how the invention also discloses combinations of preferred features: the polycondensate (A) contains repeating units derived from a dicarboxylic acid (a), a diol (b), and an ethylenically unsaturated diacid (c). These repeating units are preferably derived from raw materials represented by Formulae (I), (II) and (III) as described below. Therefore, in the context of the present invention, a polycondensate (A) containing repeating units derived from components of Formulae (I), (II) and (III) is also disclosed.

In the context of the present application, multiple limitations are to be considered in a cumulative manner.

The term **"curing"** is used herein to characterize the condensation-type cross-linking reaction described in Section 6 below.

Unless specified otherwise, references to **"dicarboxylic acid"** in the present application are intended to refer to the respective dicarboxylic acid and the corresponding mono- or di- C₁₋₈-alkyl esters and preferably the mono- or di-C₁₋₄- alkyl esters.

If the following description provides **relative amount indications** for two or more components of the same composition, wherein the specified upper limits add up to more than 100%, the indication is to be understood such that one or more of the individual amount indications contains a further implicit restriction to those relative amounts, which add up to 100% together with the remaining amount indications.

### 5.2 Polycondensate (A)

### 5.2.1 Raw materials

The **polycondensate (A)** as reported herein is obtained by reaction of raw materials comprising at least one dicarboxylic acid (a), at least one diol (b), and at least one ethylenically unsaturated diacid (c). One or more optional components (d) can also be included.

### Dicarboxylic acid (a):

In principle, any dicarboxylic acid can be used as component (a), which is capable of undergoing polycondensation reaction with diol components, provided that the ethylenically unsaturated dicarboxylic acids specified as component (c) below are excluded. The dicarboxylic acids suitable for use in the present invention are preferably characterized by the following general formula (I):

R^{a}OOC-R¹-R²-R³-COOR^{b} (I)

wherein R¹ is absent or selected from C₁₋₁₅ alkylene, C₁₁₋₁₅ alkenylene, and C₂₋₁₅ alkynylene; R² is selected from C₁₋₁₂ alkylene, C₁₁₋₁₂ alkenylene, and C₂₋₁₂ alkynylene, each of which may contain one or two 5-7-membered rings, which may optionally be condensed; R³ is absent or selected from C₁₋₁₅ alkylene, C₁₁₋₁₅ alkenylene, and C₂₋₁₅ alkynylene. Each of these groups may be unsubstituted or substituted by 1-3 substituents, wherein said substituents neither contain hydroxyl nor carboxyl or carboxylic acid ester groups and are preferably selected from C₁₋₈ alkyl, C₂₋₈ alkynyl, C₁₋₆ alkoxy. Each of R^{a} and R^{b} is independently selected from hydrogen, alkyl groups having 1 to 8 carbon atoms. One or more dicarboxylic acids (a) may be used either singly or in combination.

In a preferred embodiment, R¹ is absent; R² is selected from C₃₋₁₀ alkylene, and C₄₋₁₀ alkenylene, each being unsubstituted or substituted by 1-3 substituents, wherein said substituents are selected from C₁₋₆ alkyl; R³ is absent. Each of R^{a} and R^{b} is independently selected from hydrogen and alkyl groups having 1 to 4 carbon atoms.

In another preferred embodiment dicarboxylic acid (a) is a dimer of a fatty acid, wherein dimerization may for instance involve a 4+2 cycloaddition to yield a 6-membered cycle. Suitable dimers are described for instance in Gubbles et al. Macromolecules (2013), 46, 3975-3984 and Vendamme et al. Macromolecules (2013), 46, 3395-3405.

Fatty acids suitable for dimerization may be selected from mono- and polyunsaturated fatty acids such as oleic acid, linoleic acid, stearidonic acid, eicosatrienoic acid, eicosatetraenoic acid, eicosapentaenoic acid, clupanodonic acid, docosahexaenoic acid, tetracosapentaenoic acid, nisinic acid, mead acid, arachidonic acid, adrenic acid or calendic acid, and/or mixtures thereof as well as palmitoleic acid, eleostearic acid, ricinoleic acid, vernolic acid, licanic acid, myristoleic acid, margaroleic acid, gadoleic acid, eicosadienoic acid and/or erucic acid or mono- or dialkyl esters and mixtures thereof. In a further preferred embodiment, the fatty acid is selected from the group consisting of a linoleic, linolenic, stearidonic, eicosadienoic, eicosatrienoic, eicosatetraenoic, eicosapentaenoic, clupanodonic, docosahexaenoic, tetracosapentaenoic, nisinic, mead, arachidonic, adrenic or calendic acid or mono- or dialkyl esters and mixtures thereof. The dimer may be partially or fully hydrogenated after dimerization.

Dimers of fatty acid are commercially available. For example, dimers of oleic acid or linoleic acid are available from Croda (The Netherlands) under the trade name PRIPOL 1009 or Pripol 1006, or Oleon (Belgium) under the trade names Radiacid 0976 or Radiacid 0975. These compounds have high purity, are fully hydrogenated, and are distilled aliphatic dimer acids. These products appear as colorless to lightly colored, clear viscous liquids. Preferred properties can be summarized as follows:

| | |
|---|---|
| Purity: | ≥95% |
| Acid value: | 194-201 mg KOH/g |

Partially hydrogenated dimers of fatty acid can be obtained for instance from Arizona Chemicals (The Netherlands), under the trade mark UNIDYME.

In another preferred embodiment in combination with any of the above or below embodiments, the dimer of fatty acid used for the polycondensation is fully hydrogenated.

### Diol (b):

In principle, any diol can be used as component (b), which is capable of undergoing polycondensation reaction with dicarboxylic acid components. Diol (b) is preferably selected from the compounds characterized by the following general formula (II):

HO-R⁴-R⁵-R⁶-OH (II)

wherein R⁴ is absent or selected from C₁₋₁₅ alkylene, C₂₋₁₅ alkenylene, and C₂₋₁₅ alkynylene; R⁵ is selected from C₁₋₁₂ alkylene, C₂₋₁₂ alkenylene, and C₂₋₁₂ alkynylene, each of which may contain one or two 5-7-membered rings, which may optionally be condensed; R⁶ is absent or selected from C₁₋₁₅ alkylene, C₂₋₁₅ alkenylene, and C₂₋₁₅ alkynylene. Each of these groups may be unsubstituted or substituted by 1-3 substituents, wherein said substituents neither contain hydroxyl or carboxyl or carboxylic acid ester groups and are preferably selected from C₁₋₆ alkyl, C₂₋₆ alkenyl, C₂₋₆ alkynyl, C₁₋₆ alkoxy. One or more diols (b) may be used either singly or in combination.

In a preferred embodiment, R⁴ is absent; R⁵ is selected from C₃₋₁₀ alkylene, and C₄₋₁₀ alkenylene, each of which may be unsubstituted or substituted by 1-3 substituents, wherein said substituents are preferably selected from C₁₋₆ alkyl; R⁶ is absent.

In another preferred embodiment, diol (b) is a dimer of a fatty alcohol, wherein dimerization may for instance involve a 4+2 cycloaddition to yield a 6-membered cycle. Another preferred diol (b) is 1,4-butanediol. A combination of both fatty alcohol dimer and 1,4-butanediol is also preferred.

Dimers of fatty alcohols are commercially available, for example, from Croda (The Netherlands) under the trade name PRIPOL 2033. This compound has high purity, is fully hydrogenated, and is a distilled aliphatic dimer alcohol. This product appears as colorless to lightly colored, clear viscous liquid. Preferred properties of this compound can be summarized as follows:

| | |
|---|---|
| Dimer %: | ≥95% |
| Hydroxyl value: | 202-212mg KOH/g |
| Acid value: | ≤0.2mg KOH/g |

A partially hydrogenated dimer of fatty alcohol can be obtained for instance from Nanjing Hongbaoli Co., Ltd (China).

In another preferred embodiment in combination with any of the above or below embodiments, the dimer of fatty alcohol used for the polycondensation is fully hydrogenated.

### Ethylenically unsaturated diacid (c):

In principle, any ethylenically unsaturated dicarboxylic acid can be used as component (c), which is capable of undergoing polycondensation reaction with diol components. Ethylenically unsaturated diacid (c) is preferably selected from the compounds characterized by the following general formula (III):

R^{c}OOC-R⁷-COOR^{d} (III)

wherein R⁷ is selected from C₁₋₁₀ alkylene, C₂₋₁₀ alkenylene, and C₃₋₁₀ alkynylene. Each of these groups may be unsubstituted or substituted by 1-3 substituents, wherein said substituents are preferably selected from C₁₋₈ alkyl, C₁₋₈ alkenyl, C₂₋₈ alkynyl, C₁₋₆ alkoxy, and -R⁸-COOR⁹ wherein R⁷ and/or at least one of its substituents is selected such that one or more carbon-carbon double bonds is/are present in the part of the molecule formed by R⁷ and its substituents, wherein such carbon-carbon double bond(s) may also be formed between a carbon atom of R⁷ and the carbon atom of the substituent, which is covalently bonded to said carbon atom of R⁷. R⁸ is C₁₋₃ alkylene, C₂₋₃ alkenylene or C₂₋₃ alkynylene and R⁹ is C₁₋₆ alkyl. Each of R^{c} and R^{d} is independently selected from hydrogen and alkyl groups having 1 to 8 carbon atoms. The one or more carbon-carbon double bonds may each independently have cis or trans (Z or E) stereochemistry.

In a preferred embodiment, R⁷ is selected from C₂₋₄ alkylene, C₂₋₄ alkenylene, and C₂₋₄ alkynylene. Each of these groups may be unsubstituted or substituted by 1-3 substituents, wherein said substituents are preferably selected from C₁₋₃ alkyl, C₁₋₃ alkenyl, C₂₋₃ alkynyl, C₁₋₄ alkoxy, and -R⁸-COOR⁹ wherein such carbon-carbon double bond may also be formed between a carbon atom of R⁷ and the carbon atom of the substituent, which is covalently bonded to said carbon atom of R⁷. R⁸ is C₁₋₃ alkylene, C₁₋₃ alkenylene or C₂₋₃ alkynylene and R⁹ is C₁₋₄ alkyl. Each of R^{c} and R^{d} is independently selected from hydrogen and alkyl groups having 1 to 8 carbon atoms.

According to another preferred embodiment, the ethylenically unsaturated dicarboxylic acid (c) is represented by general formula (IIIa):

R^{c}OOC-R¹⁰-R¹¹-R¹²-COOR^{d} (IIIa)

wherein R^{c} and R^{d} are defined as above for formula (III) and wherein R¹⁰, R¹¹ and R¹² are each independently selected from the group consisting of a covalent bond, -(CR^{e}R^{f})ₘ-, -C(R^{e})=C(R^{f})- and -C(=CR^{e}R^{f})-, wherein R^{e} and R^{f} are each independently selected from C₁₋₄ alkyl, C₂₋₄ alkenyl, C₂₋₄ alkynyl, C₁₋₄ alkoxy, and -R¹³-COOR¹⁴, m represents an number of from 1 to 3, R¹³ represents C₁₋₃ alkylene, C₂₋₃ alkenylene or C₂₋₃ alkynylene and R¹⁴ represents C₁₋₄ alkyl, with the proviso that at least one of R¹⁰, R¹¹ and R¹² represents a group selected from -C(R^{e})=C(R^{f})- and -C(=CR^{e}R^{f})-.

Particularly preferred compounds (c) are selected from the group consisting of fumaric acid, maleic acid, itaconic acid, citraconic acid mesaconic acid and aconitic acid, and/or their respective C₁₋₄dialkyl esters.

One or more ethylenically unsaturated diacid (c) may be used either singly or in combination.

### Optional further components (d):

Further optional components may for instance be compounds containing one or more hydroxyl groups and one or more carboxyl groups. Bifunctional compounds of this type such as lactic acid could serve to replace simultaneously dicarboxylic acid compounds (a) and diol compounds (b). Compounds having more than two functional groups could have the same effect of replacing both components (a) and (b) and, additionally, they could also be used to introduce branching into the polycondensate. Typical compounds of this type are citric acid and citric acid ester.

Branching may also be introduced using optional components (d) that have three or more functional groups of the same type. Typical representatives of this class of optional components (d) are glycerol and pentaerythritol.

Another group of optional further components is the group of polyalkylene glycols including especially polyethylene glycol (PEG), poly(tetramethylene ether)glycol (PTMEG), and polypropylene glycol (PPG). The polyalkylene glycol for use as optional further component (d) in the present invention has a weight average molecular weight of 2000 g/mol or less. It is preferably characterized by the following general formula (IV):

H-(O-R¹⁵)ₙ₁-OH (IV)

wherein R¹⁵ represents an alkylene group having between 2 and 4 carbon atoms, including especially ethylene groups (-CH₂-CH₂-), propylene groups (-CH₂-CH₂-CH₂-), methyl-substituted ethylene (-CH(Me)-CH₂-, wherein Me represents a methyl group), tetramethylene (-CH₂-CH₂-CH₂-CH₂-), methyl-substituted propylene (-CH(Me)-CH₂-CH₂- or -CH₂-CH(Me)-CH₂-), dimethyl-substituted ethylene (-CH(Me)-CH(Me)- or -C(Me)₂-CH₂-) or mixtures thereof, with ethylene groups being preferred (polyethylene glycol, PEG); and wherein n1 is an average value and it is >1. Preferably, n1 is in the range of from 2 to 35, more preferably from 3 to 27, even more preferably from 4 to 23 and most preferably from 4 to 15. For the preferred embodiment of PEG, this means that the preferred weight-average molecular weight of component (c) is from 100 to 1500 g/mol, more preferably 150 to 1200 g/mol, even more 180 to 1000 g/mol and most preferably from 190 to 650 g/mol. Particularly preferred is the use of any one of the commercially available products PEG 200, PEG 400, PEG 600, PEG 800, PEG 1000 and PEG 1500 and any mixtures thereof. It is also possible to use different grades of the product, including industrial grade products and highly pure analytical grade products.

When using polyalkylene oxides other than PEG, the average number of repeating units should be the same. Hence, the molecular weight ranges and preferred molecular weight ranges given for PEG may be adapted accordingly, taking the differences in molecular weight of each repeating unit into account.

Yet further optional components may be higher aggregates, such as trimers or oligomers, that may be formed in the course of the above-mentioned dimerization reactions yielding dimers of fatty acids and dimers of fatty alcohols.

For the avoidance of doubt, if a component could possibly be understood to fall within the scope of two or more of the above categories (a), (b), (c) and (d), for the purpose of defining the present invention, it shall belong solely to the category mentioned earlier in the above list. For instance, some ethylenically unsaturated compounds with more than two carboxylic acid or ester groups are covered already by the definition of the above component (c). These compounds must therefore not be regarded as components (d) but only as components (c).

### Compositional ratios:

In a preferred embodiment in combination with any of the above or below embodiments, the molar ratio of component diols (or combination of diols) (b) to component diacid (or combination of diacids) (a) and (c) is from 2:1 to 1:2, more preferably from 1.3:1 to 1:1.3.

It is even more preferred to use a ratio of diol components to dicarboxylic acid components (a) and (c) such that the molar ratio of diols (b) to dicarboxylic acid (a) and (c) is either in the range of from 0.83 to 0.97 or in the range of from 1.03 to 1.17. An excess of diol component or dicarboxylic acid component results in polycondensate having a lower molecular weight and high hydroxyl or carboxylic acid functional groups respectively.

In a preferred embodiment in combination with any of the above or below embodiments, the molar ratio of component dicarboxylic acid (a) to ethylenically unsaturated diacid (c) is from 1:9 to 9:1, more preferably from 1:3 to 3:1.

In a preferred embodiment in combination with any of the above or below embodiments, the optional further component (d) is present in a concentration of 1 mol% to 60 mol% and preferably below 25 mol% relative to the total molar amount of the monomers (a) + (b) + (c) + (d) of the feed composition for making the polycondensate. If optional component (d) is present, the molar amounts of hydroxyl groups and/or carboxyl groups contained in component (d) are also to be taken into account such that the above ratios are applied in the following manner:

The molar ratio of hydroxyl groups derived from component diol (or combination of diols) (b) and component (d) to the molar amount of carboxyl groups derived from the dicarboxylic acid (or combination of dicarboxylic acids) components (a), (c) and (d) is from 2:1 to 1:2, more preferably from 1.3:1 to 1:1.3 and even more preferably in the range of from 0.83 to 0.97 or in the range of from 1.03 to 1.175.

### 5.2.2 Polycondensation

It is preferred to carry out the polycondensation without solvent, *i.e.* as a bulk condensation. The stirring speed is preferably in the range of from 120 to 250 rpm, more preferably 150 to 220 rpm. The reaction mixture is preferably heated to a temperature of 160 to 210°C, more preferably 170°C to 200°C. Due to the volatile nature of compound (c), the polycondensation is first carried out at normal pressure under a nitrogen stream for 3 to 30 hours, preferably 7 to 20, and then vacuum is applied. It is particularly preferred to carry out second part of the polycondensation in two stages, wherein the first stage involves a polycondensation at a lower vacuum of about 7-13 mbar and a temperature of about 140 to 180°C, and wherein the second stage involves polycondensation at a higher vacuum of about 2-8 mbar and a temperature of about 180-210°C. It is further preferred that the first stage has a duration of 1-3 hours and the second stage has a duration of 2-5 hours. The experimental details given in this section have been optimized for laboratory scale processes. If the process is to be carried out at a larger (e.g., industrial) scale, its process parameters need to be adjusted in an appropriate manner. Up-scaling of chemical processes can be done by a chemical engineer relying on common general knowledge and routine optimization.

In a preferred embodiment in combination with any of the above or below embodiments the polycondensation is performed in the presence of a catalyst. Catalysts that may be used for the polycondensation process are well known in the art.

In another preferred embodiment in combination with any of the above or below embodiments, the esterification catalyst is selected from the group consisting of salts and oxides of Li, Ca, Mg, Mn, Zn, Pb, Sb, Sn, Ge, and Ti, and their glycol adducts, more preferably acetate salts of Li, Ca, Mg, Mn, Zn, Pb, Sb, Sn, Ge, and Ti, and Ti alkoxides, even more preferably titanium(IV) n-butoxide, tin(II) octoate, butyl tin chloride dihydroxide, manganese acetate, or zinc acetate, in particular titanium(IV) n-butoxide.

In a preferred embodiment in combination with any of the above or below embodiments, the polymerization catalyst is used in a concentration of 0.001 to 2 wt%, more preferably 0.005 to 0.5 wt%, in particular about 0.02 wt% with respect to the total weight of monomers (a) to (d) in the feed composition. It is preferred to employ the lowest possible amount of catalyst that yields a satisfactory yield at an acceptable reaction time. Using more catalyst than necessary will lead to darker polycondensates. The minimum required amount of catalyst can be determined by systematic routine optimization procedures.

In a more preferred embodiment the polymerization is performed in presence of titanium(IV) n-butoxide as catalyst. More preferably, the catalyst, such as titanium(IV) n-butoxide, is present at concentration comprised between 0.001 wt% to 1 wt% with respect to the weight of diacid.

In a preferred embodiment in combination with any of the above or below embodiments, the catalyst is added at the beginning of the reaction. In another preferred embodiment in combination with any of the above or below embodiments, the catalyst is added one or more times to the reaction mixture during the reaction period.

In a preferred embodiment in combination with any of the above or below embodiments, the polycondensation reaction is conducted in the presence of a radical inhibitor to prevent reaction of the ethylenic insaturations during the polyester formation. Inhibitors that may be used for this purpose are well known in the art.

In another preferred embodiment in combination with any of the above or below embodiments, the inhibitor is selected from the group consisting of hydroquinone, 4-methoxyphenol (also known as monomethyl ether hydroquinone or MEHQ), 2-methylhydroquinone, benzoquinone or 2-methylbenzoquinone.

In a preferred embodiment in combination with any of the above or below embodiments, the inhibitor is added simultaneously with the ethylenically unsaturated diacid (c).

In a preferred embodiment in combination with any of the above or below embodiments, the radical inhibitor is used in a concentration of 5 to 800 ppm, more preferably 50 to 300 ppm with respect to the total monomer feed composition.

### 5.2.3 Molecular Weight and Molecular Weight distribution

The polycondensate (A) is typically characterized by a molecular weight of from 35,000 to 95,000 g/mol, preferably from 45,000 to 70,000 g/mol. It is furthermore preferable that the polycondensate (A) exhibits a molecular weight distribution Mw/Mn of from 1.00 to 3.5, preferably from 1.5 to 2.8.

The adhesive level is influenced by the polymer molecular weight, and a molecular weight greater than 45,000 g/mol, preferably from 51,000 g/mol to 90,000 g/mol is preferred in order to get good adhesion on human skin. Furthermore such a high molecular weight is preferable since it allows the incorporation of a higher amount of oil with the adhesive structure.

If the desired molecular weight cannot be reached by direct polycondensation, the molecular weight of the polymer can be increased *via* chain extension reaction. Suitable compounds for the chain extension for OH terminated polycondensate are difunctional isocyanate compounds such as hexamethylene 1,6-diisocyanate, tolulene diisocyanate, m-phenylene diisocyanate, p-phenylene diisocyanate, xylylene diisocyanate, 4,4'-diphenyl methane diisocyanate, 2,4'-diphenyl methane diisocyanate, 2,2'-diphenyl methane diisocyanate, hexamethylene diisocyanate, isophorone diisocyanate, polymethylenepolyphenyl diisocyanate, 3,3'-dimethyl-4,4'-biphenylene diisocyanate, 3,3'-dimethyl-4,4'-diphenylmethane diisocyanate, 3,3-dichloro-4,4'-biphenylene diisocyanate or 1,5-naphthalene diisocyanate. For carboxyl-terminated polycondensates, carbodiimides are suitable for use as chain extenders. Suitable chain extender compositions containing a difunctional carbodiimide that may be used for chain extension purposes are commercially available, for example, from BASF (Germany) under the trademark Lupranate.

In a preferred embodiment in combination with any of the above or below embodiments, the chain extension reaction is performed in presence of heat and/or a catalyst. Catalysts that may be used for the polycondensation process are well known in the art.

The preferred concentration range for chain extension agents is from 3 to 12 mol% and more preferably 3 to 9 mol% (with respect to polycondensate molar amount). Chain extension using chain extension agents can be performed by mixing the polycondensate and chain extension agent optionally in presence of a catalyst and/or in the optional presence of heat. Catalysts suitable for use in this reaction are well-known to the person skilled in the art. They include especially zirconium (IV) acetylacetonate, which is preferably used at a concentration from 0.001 to 2 wt% and most preferably 0.01 to 0.1 wt% (with respect to polycondensate weight).

### 5.2.4 Further Properties

The polycondensate (A) is preferably characterized by one or more of the following further properties:
- α transition temperature below 0°C, preferably from -80°C to -20°C, even more preferably from -60°C to -45°C.
For polycondensate having a molar ratio of diols (b) to diacid (a) and (c) is in the range of from 1.03 to 1.17 (*i.e.* hydroxyl terminated polycondensate):
- acid value (expressed in mg KOH/g polymer) from 0.1 to 150, preferably from 1 to 20, even more preferably from 0.1 to 1.
- hydroxyl value (expressed in mg KOH/g polymer) from 1 to 150, preferably from 1 to 50, even more preferably from 15 to 25.
For polycondensate having a molar ratio of diols (b) to diacid (a) and (c) is in the range of from 0.83 to 0.97 (*i.e.* carboxylic acid terminated polycondensate):
- acid value (expressed in mg KOH/g polymer) from 1 to 150, preferably from 1 to 80, even more preferably from 5 to 40.
- hydroxyl value (expressed in mg KOH/g polymer) from 0.11 to 150, preferably from 0.1 to 50, even more preferably from 0.1 to15.

### 5.3 Oil additive (B)

The composition of the present invention optionally contains an oil additive (B). Said oil additive may be selected from any natural or synthetic, organic or mineral oil which is suitable for topical administration to the human skin. The oil additive may also be selected from the group of softeners and plasticizers employed in the polymer industry, provided the softener or plasticizer is suitable for topical administration to the human skin. In a preferred embodiment the oil additive is colourless, and its viscosity is lower than that of the polycondensate. It is also preferred that the polycondensate is fully soluble in the oil additive or in the mixture of the oil additive and an organic solvent.

If present, the amount of the oil additive (B) in the composition is preferably from 0.01 to 1.5 parts by weight, preferably 0.2 to 1.2 parts by weight, more preferably 0.5 to 1 parts by weight, based on 1 part by weight of the polycondensate (A) before cross-linking.

In another preferred embodiment, and in combination with any of the above or below embodiments, the oil additive is bio-based.

In another preferred embodiment, and in combination with any of the above or below embodiments, the oil additive does not contain any free carboxylic acid (either as part of the additive or as an impurity) in order to reduce risk of skin irritation.

In another preferred embodiment, and again in combination with any of the above or below embodiments, the oil additive does not contain unsaturation. The absence of unsaturated groups is helpful in order to avoid undesired side reactions, and "yellowing" effects if the adhesive is sterilized. Aromatic ring-containing chemical structures are also not preferable.

The oil additive can for instance be one or more oils selected from the following list, but it is not limited thereto: sorbitan mono- or di-esters of fatty acids such as sorbitan ester of oleic acid, oleate esters, soybean oil, citrate ester, vernonia oil, PEG (Mw 100-1000) and PEG derivatives such as PEG ether (such as dimethyl PEG) or-PEG esters such as PEG distearate, fatty acids such as myristic acid, palmitic acid, stearic acid, lauric acid, oleic acid, isostearic acid, neodecanoic acid, trimethylhexanoic acid and neoheptanoic acid; the esters of these fatty acids such as isopropyl myristate; fatty alcohols such as myristyl alcohol, cetyl alcohol, oleyl alcohol and lauryl alcohol; aliphatic pyrrolidones such as *N*-lauryl-2-pyrrolidone; terpenes such as 1-menthol, d-limonene and α-terpineol; alkanes such as heptane, octane, nonane and decane; and substances acting as solubilizers and transdermal absorption promoters of medicinal ingredients such as crotamiton and α -, β - and γ -cyclodextrin. Besides, mention may be made of substances for use as fragrant/refreshing materials such as L-menthol, camphor, thymol, mint oil, castor oil, fennel oil, star anise oil, cinnamon oil, oil of cloves, thiamine oil, turpentine oil, eucalyptus oil, lavender oil, lemon oil, orange oil, bergamot oil and rose oil. Additionally, fatty acid esters of monovalent alcohols such as cetyl octanoate, hexyl laurate, isopropyl myristate, isopropyl palmitate, butyl stearate, myristyl lactate, and the like; dibasic acid esters such as dioctyl adipate, diethyl sebacate, dioctyl sebacate, dioctyl succinate, and the like; fatty acid esters of polyvalent alcohols and the like such as propylene glycol dicaprate, glycerol trioctanoate, glycerol tri(octanoate/decanoate), medium chain fatty acid triglyceride, and the like; and in particular, isopropyl myristate, isopropyl palmitate, diethyl sebacate, middle chain fatty acid triglyceride, and the like are preferably used. Further examples of the oil additive include glycols such as ethylene glycol, diethylene glycol, propylene glycol, dipropylene glycol, tripropylene glycol, triethylene glycol, poly(propylene glycol) and the like; fats and oils such as olive oil, castor oil and the like; lanolin; hydrocarbons such as squalane and liquid paraffin; various surfactants; ethoxylated stearyl alcohol; glycerol monoesters such as oleic acid monoglyceride, caprylic acid monoglyceride and lauryl acid monoglyceride; dialkyl ester of polyalkylene glycol such as poly(propylene glycol); glycerol diester such as glycerol diacetate and the like, glycerol triester such as glycerol triacetate and the like, or a mixture thereof; fatty acid alkyl ester such as triethyl citrate and the like; long chain alcohol; higher fatty acid such as oleic acid and caprylic acid; alkyl ester of higher fatty acid such as isopropyl myristate; pyrrolidones such as N-methylpyrrolidone and N-dodecylpyrrolidone; sulfoxides such as decyl methyl sulfoxide; 1,3-butanediol and the like. All of the above oil additives can be used alone or in a mixture of two or more kinds thereof.

In another preferred embodiment, and in combination with any of the above or below embodiments, the oil additive is a isosorbide ester (monoester or diester) or a mixture of two or more isosorbide esters, each of which being selected from isosorbide monoesters or isosorbide diesters. The isosorbide diester employed can be both symmetrical or asymmetrical. Preferably, isosorbide is esterified with one or two fatty acids, both being independently selected from C6-18 saturated or unsaturated fatty acids, more preferably C8-12 saturated or unsaturated fatty acids. Isosorbide dicaprylate is commercially available from Sytheon under the trade mark Synovea^{®} DOI. A mixture of isosorbide diesters is commercially available under the trade mark POLYSORB ID 37 (Roquette Frères, France). As an alternative to esters of isosorbide, it is also possible to employ the esters of stereoisomers of isosorbide, namely esters of isomannide or isoidide. However, it is more preferably to employ isosorbide due to its commercial availability and low cost.

Among the above-mentioned oil components, it is particularly preferred to employ one or more oil components selected from the group of sorbitan mono- or di-esters of fatty acids, PEG diesters, sesame oil, POLYSORB ID37, sorbitol mono- and diesters as well as isopropyl myristate.

For the purposes of this invention, where the composition or adhesive composition is to be used for medical purposes, for example as part of a medical tape or patch, then the oil additive (B) is preferably included.

### 5.4 Tackifier (C)

In a preferred embodiment, and in combination with any of the above or below embodiments, the composition of the invention or adhesive contains a tackifier agent or tack enhancer agent or a combination of two or more of such agents (hereinafter jointly referred to as "tackifier"). If present, the tackifier (C) is preferably in an amount from 0.001 to 0.3 parts by weight, preferably 0.01 to 0.05 parts by weight, based on 1 part by weight of the polycondensate (A) before cross-linking.

In another preferred embodiment in combination with any of the above or below embodiments, the tackifier agent is bio-based.

The tackifier can for instance be rosin or a rosin derivative, but it is not limited to this type of substances. The tackifier is preferentially selected from the group consisting of rosins and their derivates (abietic acid, abietic acid esters), terpenes and their derivatives (polyterpene resins, terpene-phenol resins), aliphatic, cycloaliphatic and aromatic resins (C5 aliphatic resins, C9 aromatic resins, and C5/C9 aliphatic/aromatic resins), hydrogenated hydrocarbon resins, and their mixtures.

Suitable rosin derivatives are for instance: esterified hydrogenated rosin such as: Foralyn 90, Foralyn 110, Foralyn 5020-F (EASTMAN); and ester of tall oil rosin such as: Sylvalite RE 105XL (Arizona Chemical - The Netherlands).

In another preferred embodiment, and in combination with any of the above or below embodiments, the tackifier does not contain free carboxylic acid (either as part of the tackifier or as an impurity) in order to reduce risk of skin irritation. In another preferred embodiment, and in combination with any of the above or below embodiments, the tackifier does not contain unsaturation. The absence of unsaturated groups is advantageous in order to avoid undesired side reactions, and "yellowing" effect if the adhesive is sterilized. Tackifiers containing aromatic rings are also not preferable.

### 5.5 Ethylenically unsaturated additive (D)

In a preferred embodiment, and in combination with any of the above or below embodiments, the composition of the invention or the adhesive contains an ethylenically unsaturated additive (D). If polycondensate (A) is formed in the composition in situ, then preferably the ethylenically unsaturated additive (D) is not added until after the polycondensation has been completed. Such an additive (D) can be used to adjust the viscosity of the adhesive composition or to facilitate the coating step.

The term "ethylenically unsaturated additive" as used herein refers to low molecular weight compounds having an ethylenically unsaturated compound that could be co-polymerised with the ethylenically unsaturated dicarboxylic acid units of the polycondensate (A) during the curing step of the adhesive. The term "low molecular weight", as used herein, denotes a molecular weight of less than 2,000 g/mol, preferably less than 500 g/mol, more preferably less than 300 g/mol. The most preferred ethylenically unsaturated additives belong to the class of mono-functional acrylic esters or methacrylic esters, where the alkyl segment is a saturated, unsaturated or aromatic hydrocarbon chain which is linear, branched or cyclic. In a preferred embodiment in combination with any of the above or below embodiments, the alkyl chain of the (meth)acrylic acid ester has from 3 to 18 carbon atoms, *i.e.* 3, 4, 5, 6, 7, 8, 9, 10, 11, 12, 13, 14, 15, 16, 17 or 18 carbon atoms, more preferably from 2 to 8 carbon atoms, *i.e.* 2, 3, 4, 5, 6, 7 or 8 carbon atoms. In another preferred embodiment in combination with any of the above or below embodiments, the ethylenically unsaturated additive is at least partially derived from renewable sources, in particular, selected from the group consisting of n-butyl acrylate, 2-ethylhexyl acrylate, or n-octyl acrylate.

In a preferred embodiment, and in combination with any of the above or below embodiments, the ethylenically unsaturated additive (D) is present in an amount from 0.01 to 0.8 parts by weight, preferably 0.05 to 0.3 parts by weight, based on 1 part by weight of the polycondensate (A) before cross-linking.

### 5.6 Optional further components of the composition

Further components may optionally be present, provided these optional further components do not interfere with the beneficial effects of the invention. Additional components suitable for use in the adhesive of the present invention include the following: one or more active pharmaceutical ingredients such as those listed herein, moisture regulating agents such as alginates; aginate derivatives and alginate salts (as for instance zinc alginate or silver alginate); starch; cellulose derivatives such as carboxymethyl cellulose (CMC), hydroxyethyl cellulose or acetate cellulose and their salts; gelatine and the like such as agar-agar or pectin; gums (such as gum arabic, guar gum, locust bean gum); collagen; polysaccharide such as chitosan, hyaluronic acid or hyaluronate derivatives and salts; activated charcoal; as well as components having antibacterial activity such as silver particles/crystal and silver salts such as silver sulfadiazine; sulfated molecules and sulfate rich polymers and iodine derivatives. It is advantageous to employ only optional further components, which do not participate in the curing reaction or, at least, which do not participate in the curing reaction to such an extent that the effects of the present invention are detrimentally affected to a significant extent.

Undesired reactions of this type between the optional further components and the chain extension agent can be prevented by adding the optional further component only after the polycondensate has been chain-extended.

In a preferred embodiment in combination with any of the above or below embodiments, the composition or adhesive comprises a filler, more preferably selected from the group consisting of a silicate, talcum, calcium carbonate, clay, carbon black, pigments and pigment pastes, or pulp chopped fibers (preferably selected from the group consisting of kenaf, hemp, flax, jute, sisal, cotton and linen).

A sensitizer may also optionally be contained to improve the radical generation efficiency of a radical initiator, and to increase the range of the photosensitive wavelengths. Preferably, a sensitizer is used, which sensitizes the radical initiator by way of an electron transfer mechanism or energy transfer mechanism is preferred.

Examples of the sensitizer, which may be used in the present invention, include the following compounds which have an absorption wavelength in a wavelength region of 300 nm to 450 nm. Examples of the sensitizer include polynuclear aromatics (for example, phenanthrene, anthracene, pyrene, perylene, triphenylene, 9,10-dialkoxyanthracene), xanthenes (for example, fluorescein, eosine, erythrosine, Rhodamine B, rose bengal), thioxanthones (isopropylthioxanthone, diethylthioxanthone, chlorothioxanthone), cyanines (for example, thiacarbocyanine, oxacarbocyanine), merocyanines (for example, merocyanine, carbomerocyanine), phthalocyanines, thiazines (for example, thionine, ethylene blue, toluidine blue), acridines (for example, acridine orange, chloroflavin, acriflavine), anthraquinones (for example, anthraquinone), squaryliums (for example, squarylium), acridine orange, coumarins (for example, 7-diethylamino-4-methylcouinarin), ketocoumarin, phenothiazines, phenazines, styryl benzenes, azo compounds, diphenyl methane, triphenyl methane, distyryl benzenes, carbazoles, porphyrin, spiro compounds, quinacridone, indigo, styryl compounds, pyrylium compounds, pyrromethene compounds, pyrazolotriazole compounds, benzothiazole compounds, barbituric acid derivatives, thiobarbituric acid derivatives, aromatic ketone compounds such as acetophenone, benzophenone, thioxanthone, Michler's ketone, and heterocyclic compounds such as N-aryloxazilidinone or the like.

The content of the sensitizer in the composition is preferably from 0.001 to 0.2 parts by weight, preferably from 0.005 to 0.15 parts by weight, based on 1 part by weight of the polycondensate (A) before cross-linking.

The sensitizer may be used alone, or may be used in combination of two or more kinds.

### 6 Adhesive and adhesive preparation

### 6.1 Overview

The adhesive of the present invention is a composition comprising the above polycondensate (A) in a cross-linked form together with an optional oil additive. Further optional components may be present as long as these do not prevent the beneficial effects of the present invention from being accomplished.

The adhesive characteristics can be fine-tuned by multiple means, such as:
- varying the concentration of component (c) in the polycondensate (A), and/or
- varying the dissipative character of the glue by tuning the ratio, structure and functionality of oil additive (B) and tackifier (C) within the adhesive composition, and/or
- varying the degree of curing of the adhesive (for instance by tuning the dose of radiation in the case of UV curing or tuning the curing temperature and/or time in the case of a thermal curing event), and/or
- varying the amount, structure and/or functionality of the ethylenically unsaturated additives (D) added to the polycondensate (A) prior curing the adhesive.

### 6.2 Cross-linking reaction

In another preferred embodiment, and in combination with any of the above or below embodiments, the method for preparing the adhesive composition comprises mixing the polycondensate (A) and the optional oil additive (B), optionally with any other components such as the tackifier (C) and ethylenically unsaturated additive (D) either by:
- Mixing two or more components in bulk (without solvent). In that case it may be preferable to heat at a temperature between 40°C to 100°C in order to decrease the melt viscosity and ensure good homogenization of the mixture, or
- Mixing at room temperature is also possible (e.g. if a thermal initiator is to be used for the cross-linking) but could require the addition of an organic solvent and/or of the ethylenically unsaturated additives (D) in order to create a reactive formulation with a suitable viscosity enabling the coating step.

The present invention further relates to a process for radically curing the composition, for example wherein the curing is effected by adding an initiator to the composition. An initiator may not be required in some cases (e.g. where curing is effected by e-beam). Preferably, the curing is effected at a temperature in the range from -20°C to +200°C and the initiator, if required, can be a photo-initiator, a thermal initiator and/or a redox initiator.

In a preferred embodiment, in combination with any of the above or below embodiments, the composition is cured by an electromagnetic irradiation initiated process.

In the electromagnetic irradiation curing method, it is preferred that the composition does not contain any organic solvents. If coating has to be done at room temperature, ethylenically unsaturated additive (D) could be advantageously added to the composition for lowering the viscosity of the resulting formulation in order to make handling (coating) thereof more easy. Alternatively, a hot-melt processing can also be applied for coating the composition. In that later case, addition of an ethylenically unsaturated additive (D) is not necessary, and the viscosity of the composition is conveniently adjusted by performing the lamination at a temperature ranging from 40°C to 120°C.

In another preferred embodiment, in combination with any of the above or below embodiments, a photo-initiating system can be a photo-initiator as such, or a mixture of photo-initiators, and can be chosen from the large numbers of photo-initiating systems known by the person skilled in the art. Suitable photoinitiators that create free radicals upon irradiation with light of respective wavelength are a preferred group of catalysts. Examples of suitable photoinitiators include the compounds manufactured by Ciba, Switzerland under the trade names Darocur^{®} and Irgacure^{®}. Such initiator compounds are usually added to the composition in small amounts, for example, 0.1 wt% to 3 wt%, preferably from 0.5 wt% to 2 wt%, relative to the total weight of the composition.

In another preferred embodiment, in combination with any of the above or below embodiments, a photo-initiating system can be a photo-initiator as such, or a mixture of photo-initiators, and can be chosen from the large numbers of photo-initiating systems known by the person skilled in the art. Suitable photo-initiators that create free radicals upon irradiation with light of respective wavelength are a preferred group of catalysts. Preferably, the photo-initiator is selected from the group consisting of diacyl peroxides, dialkylperoxidicarbonates, tert-alkyl peroxyesters, di-tert-alkyl peroxides, teralkylhydroperoxides, ketone peroxides, and mixtures thereof. Examples of suitable photoinitiators include the compounds manufactured by Ciba, Switzerland under the trade names Darocur^{®} and Irgacure^{®}. Such initiator compounds are usually added to the composition in small amounts, for example, 0.1 wt% to 10 wt%, preferably from 0.5 wt% to 2 wt%, relative to the polycondensate weight. The absorption characteristics of the photoinitiator are not particularly limited. It is possible to freely choose a photoinitiator or a mixture of photoinitiators having absorbance characteristics that match the intended type of curing irradiation. If the composition contains an optional component that has a strong absorbance in UV, a photoinitiator with different wavelength absorbance profile should be selected. This comment is valid for all optional components that can be used in connection with the present invention.

According to one embodiment, curing is initiated by electromagnetic irradiation. Said electromagnetic irradiation may be in any wavelength or range of wavelengths, for which a suitable photoinitiator or system of photoinitiator and sensitizer is available. This may include gamma-rays, X-rays, UV rays as well as visible light.

In another preferred embodiment, in combination with any of the above or below embodiments, the adhesive is cured by a thermally-initiated process. The thermal initiator can be selected from azo compounds like for example azoisobutyronitril (AIBN), C-C labile compounds like for example benzopinacole, peroxides, and mixture thereof. In the thermal-curing method, the composition could contain both an organic solvent and an ethylenically unsaturated additive.

In another preferred embodiment, in combination with any of the above or below embodiments, the adhesive is cured by a redox-initiated process. The redox initiator can be selected from hydroperoxides, peroxycarbonates, peroxyesters, diacylperoxides, or dialkylperoxides. In the redox-curing method, the composition could contain both an organic solvent and an ethylenically unsaturated additive.

In another preferred embodiment, in combination with any of the above or below embodiments, the adhesive is cured via e-beam process. In this particular case the use of initiator is optional.

### 6.3 Cross-linked polycondensate

In a preferred embodiment in combination with any of the above or below embodiments, the adhesive gel content value of the cross-linked polycondensate in the adhesive composition is from 40 % to 95%, most preferably from 60 % to 80%.

### 6.4 Manufacture of the adhesive of the invention

The method for manufacturing the adhesive of the invention is not particularly limited. In a preferred embodiment of practicing the invention, a reaction mixture is formed by mixing the polycondensate (A) with the optional oil additive (B), a solvent (optional) or a mixture of solvents (optional), the tackifier (optional), the ethylenically unsaturated additive (optional) and the photoinitiator as well as any further optional components. If the tackifier is a solid, a solution of tackifier in a compatible solvent is preferably used. Once the reaction mixture is homogeneous, the solution is casted on a liner and cured by means of the cross-linking reaction described herein. After curing, the "free" side of the adhesive, that is, the side not in contact with the liner, may be laminated onto a semi-finished product, such as a backing, support, protective film or other product, to thereby yield the final product of interest. Alternatively, the "free" side of the adhesive may be simply laminated onto a further liner surface for storage purposes.

It may furthermore be advantageous to mix the polycondensate (A) initially with oil to decrease the viscosity before adding further ingredients. It is also preferable that the photoinitiator is added just before coating and curing. The tackifier may be added together with the oil or after the oil.

### 7. Medical Tape and Patch

### 7.1 Backing

To prepare a medical tape or patch, the adhesive is laminated on a backing. Suitable backing materials may, for instance, be selected from woven and non-woven materials, wherein preferred woven backing materials are selected from cotton, nylon, rayon or a mixture thereof; and wherein preferred non-woven materials are preferably selected from polyethylene terephthalate (PET), polyethylene (PE), polyurethane (PU), polyvinyl chloride (PVC), polypropylene (PP), wood pulp, cellulose, starch and/or polylactic acid (PLA). It is advisable to choose a backing material that exhibits a high degree of transparency, e.g. above 50%, preferably above 75%, for the electromagnetic wavelength region, which is contemplated for initiation of curing.

In a preferred embodiment in combination with any of the above or below embodiments the adhesive is laminated onto a bio-based non-woven having one or more of the following properties: weight: 15 - 70 g/m², thickness: 0.10 - 1.0 mm, maximum tensile strength: 70 - 120N/50mm and maximum elongation 15-85%. It is more preferred to use a backing material fulfilling two of these properties, even more preferably three of these properties and most preferably all four of these properties.

In a preferred embodiment the backing material used can withstand radiation induced curing of the adhesive formulation without visible and/or mechanical change.

In a preferred embodiment the backing material used can withstand sterilization process without visible changes (e.g. no yellowing).

### 7.2 Patch-related Features

The patch of the present invention has a construction and layer structure corresponding to that of a conventional patch. It is characterized by the presence of the adhesive of the present invention instead of the presence of a standard pressure-sensitive adhesive.

Hence, the patch of the present invention may comprise one or more of the following features: structure consisting of non-adhesive area and adhesive area; structure comprising non-adhesive fluid absorbent material and adhesive area; structure comprising moisture regulation material/compound and adhesive area; structure containing active agent reservoir material and adhesive area.

The present invention also encompasses patches, wherein adhesive thickness is not the same everywhere in the patch. This can be, for instance, patches, wherein the adhesive is thicker or thinned at the edge.

The present invention also encompasses patches that contain an active pharmaceutical ingredient, which is to be administered to the.patient by transdermal or topical delivery.

The active pharmaceutical ingredient to be delivered is not particularly limited. It is for instance possible to incorporate an active pharmaceutical ingredient selected from one of the following classes of drugs (it will be appreciated that some of biologically active compounds can be classified in more than one of these classes):

Cardiovascular drugs, in particular antihypertensive agents (e.g. calcium channel blockers or calcium antagonists) and antiarrhythmic agents; congestive heart-failure pharmaceuticals; inotropic agents; vasodilators; ACE inhibitors; diuretics; carbonic anhydrase inhibitors; cardiac glycosides; phosphodiesterase inhibitors; a blockers; [beta]-blockers; sodium channel blockers; potassium channel blockers; [beta]-adrenergic agonists; platelet inhibitors; angiotensin II antagonists; anticoagulants; thrombolytic agents; treatments for bleeding; treatments for anaemia; thrombin inhibitors; antiparasitic agents; antibacterial agents; insulin; human growth hormone and peptides; vaccines; antiinflammatory agents, in particular non-steroidal antiinflammatory agents (NSAIDs), more particularly COX-2 inhibitors; steroidal antiinflammatory agents; prophylactic antiinflammatory agents; antiglaucoma agents; mast cell stabilisers; mydriatics; agents affecting the respiratory system; allergic rhinitis pharmaceuticals; alpha-adrenergic agonists; corticosteroids; chronic obstructive pulmonary disease pharmaceuticals;xanthine-oxidase inhibitors; antiarthritis agents; gout treatments; autacoids and autacoid antagonists; antimycobacterial agents; antifungal agents; antiprotozoal agents; anthelmintic agents; antiviral agents especially for respiratory, herpes, cyto-megalovirus, human immunodeficiency virus and hepatitis infections; treatments for leukemia and kaposi's sarcoma; pain management agents in particular opioids, anaesthetics and analgesics; neuroleptics; sympathomimetic pharmaceuticals; adrenergic agonists; drugs affecting neurotransmitter uptake or release; anticholinergic pharmaceuticals; antihaemorrhoid treatments; agents to prevent or treat radiation or chemotherapeutic effects; liopgenisis drugs; fat reducing treatments; anti-obesity peptides; antiobesity agents such as lipase inhibitors; sympathomimetic agents; treatments for gastric ulcers and inflammation such as proton pump inhibitors; prostaglandins; VEGF inhibitors; antihyperlipidemic agents, in particular statins; drugs that affect the central nervous system (CNS) such as antipsychotic, antiepileptic and antiseizure drugs (anticonvulsants), psychoactive drugs, stimulants, antianxiety and hypnotic drugs, antidepressant drugs; anti-Parkinson's pharmaceuticals; hormones and fragments thereof such as sex hormones; growth hormone antagonists; gonadotropin releasing hormones and analogues thereof; steroid hormones and their antagonists; selective estrogen modulators; growth factors; antidiabetic pharmaceuticals such as insulin, insulin fragments, insulin analogues, glucagon-like peptides and hypoglycaemic agents; H1, H2, H3 and H4 antihistamines; peptide, protein, polypeptide, nucleic acids and oligonucleotide pharmaceuticals; analogues, fragments and varients of natural proteins, polypeptides, oligonucleaotides and nucleic acids and such like compounds; agents used to treat migraine headaches; asthma pharmaceuticals; cholinergic antagonists; glucocorticoids; androgens; antiandrogens; inhibitors of adrenocorticoid biosynthesis; osteoporosis treatments such as biphosphonates; antithyroid pharmaceuticals; suncreens, sun protectants and filters; cytokine agonists; cytokine antagonists; anticancer drugs; anti-Alzheimer drugs; HMGCoA reductase inhibitors; fibrates; cholesterol absorption inhibitors; HDL cholesterol elevating agents; triglyceride reducing agents; antiageing or antiwrinkle agents; precursor molcules for the generation of hormones; proteins such as collagen and elastin; antibacterial agents; anti acne agents; antioxidants; hair treatments and skin whitening agents; suncreens, sun protectants and filters; variants of human apolipoprotein; precursor molecules for generation of hormones; proteins and peptides thereof; amino acids; plant extracts such as grape seed extract; DHEA; isoflavones; nutritional agents including vitamins, phytosterols and iridoid gylcosides, sesquiterpene lactones, terpenes, phenolic glycosides, triterpenes, hydroquinone derivatives, phenylalkanones; antioxidants such as retinol and other retinoids including retinoic acid and co-enzyme Q10; omega-3-fatty acids; glucosamine; nucleic acids, oligonucleotides, antisense pharmaceuticals; enzymes; cytokines; cytokine analogues; cytokine agonists; cytokine antagonists; immunoglobulins; antibodies; antibody pharmaceuticals; gene therapies; lipoproteins; erythropoietin; vaccines; small and large molecule therapeutic agents for the treatment, or prevention of human and animal diseases such as allergy/asthma, arthritis, cancer, diabetes, growth impairment, cardiovascular diseases, inflammation, immunological disorders, baldness, pain, ophthalmological diseases, epilepsy, gynaecological disorders, CNS diseases, viral infections, bacterial infections, parasitic infections, GI diseases, obesity, and haemological diseases. Some specific non-limiting examples of suitable biologically active compounds include: anaesthetics: including amino-ester and amino-amide anaesthetics such as benzocaine, chloroprocaine, cocaine, reserpine, guanethidine, cyclomethycaine, dimethocaine/larocaine, propoxycaine, procaine/novocaine, proparacaine, tetracaine/amethocaine; articaine, bupivacaine, carticaine, cinchocaine/dibucaine, etidocaine, levobupivacaine, lidocaine/lignocaine, mepivacaine, piperocaine, prilocaine, ropivacaine, trimecaine, propofol, halothane, enflurane barbiturates, benzodiazepines, neostigmine and ketamine alkylating agents: including carmustine, cyclophosphamide, ifosfamide, streptozotocin and mechlorethamine calcium channel blockers: including amlodipine, aranidipine, azelnidipine, barnidipine, benidipine, cilnidipine, clevidipine, cronidipine, darodipine, dexniguldipine, efonidipine, elnadipine, elgodipine, felodipine, flordipine, furnidipine, iganidipine, isradipine, lacidipine, lemildipine, lercanidipine, manidipine, mesuldipine, nicardipine, nifedipine, niludipine, nilvadipine, nimodipine, nisoldipine, nitrendipine, olradipine, oxodipine, palonidipine, pranidipine, sagandipine, sornidipine, teludipine, tiamdipine, trombodipine, watanidipine, verapamil, gallopamil, benzothiazepine, diltiazem, mibefradil, bepridil, fluspirilene and fendiline antiarrhythmic and antiangina agents: including amiodarone, disopyramide, flecainide acetate, quinidine sulphate, nitroglycerine, ranolazine, amiodarone, isosorbide and alteplase antibacterial, antibiotic and antiacne agents: including amoxicillin, ampicillin, azithromycin, benethamine penicillin , bleomycin, benzoyl peroxide, cinoxacin, chloramphenicol, daunorubicin, plicamycin, fluoroquinolones, ciprofloxacin, clarithromycin, clindamycin, clindesse, clofazimine, chlorohexidine gluconate, cloxacillin, demeclocycline, doxycycline, erythromycin, ethionamide, imipenem, indomethacin, lymocycline, minocycline, nalidixic acid, nitrofurantoin, penicillin, rifampicin, spiramycin, sodium sulfacetamide, sulphabenzamide, sulphadoxine, sulphamerazine, sulphacetamide, sulphadiazine, sulphafurazole, sulphamethoxazole, sulphapyridine, tetracycline, cephalexin, cefdinir, triclosan, ofloxacin, vancocin, glyburide, mupirocin, cefprozil, cefuroxime axetil, norfloxacin, isoniazid, lupulone, D-penicillamine, levofloxacin, gatifoxacin, and trimethoprim anticancer: including doxorubicin, 6-thioguanine, paclitaxel, docetaxel, camptothecin, megestrol acetate, navelbine, cytarabine, fludarabine, 6-mercaptopurine, 5-fluorouracil, teniposide, vinblastine, vincristine, cisplatin, colchicine, carboplatin, procarbazine and etopside antidepressants, antipsychotics and antianxiety: including alprazolam, amoxapine, bentazepam, bromazepam , clorazipine, clobazam, clotiazepam, diazepam, lorazepam, flunitrazepam, flurazepam, lormetazepam, medazepam, nitrazepam, oxazepam, temazepam, maprotiline, mianserin, nortriptyline, risperidone, sertraline, trazodone, baloperidol, trimipramine maleate fluoxetine, ondansetron, midazolam, chlorpromazine, haloperidol, triazolam, clozapine, fluopromazine, fluphenazine decanoate, fluanisone, perphenazine, pimozide, prochlorperazine, sulpiride, thioridazine, paroxitine, citalopram, bupropion, phenelzine, olanzapine, divalproex sodium and venlafaxine tricyclics: including azothiopine, amitriptyline, famotidine, promethazine, paroxatine, oxcarbazapine and mertazapine antidiabetics: including acetohexamide, chlorpropamide, glibenclaraide, gliclazide, glipizide, metformin, tolazamide, glyburide, glimepiride and tolbutamide antiepileptics: including beclamide, carbamazepine, gapapentin, tiagabine, vigabatrin, topiramate, clonazepam, ethotoin, methoin, methsuximide, methylphenobarbitone, oxcarbazepine, paramethadione, phenacemide, phenobarbitone, phenyloin, phensuximide, primidone, sulthiamine, phenytoin sodium , nirofurantoin monohydrate, gabapentin, lamotrigine, zonisamide, ethosuximide and valproic acid hypnotics/sedatives and muscle relaxants: including Zolpidem tartrate, amylobarbitone, barbitone, butobarbitone, pentobarbitone, brotizolam, carbromal, chlordiazepoxide, chlormethiazole, ethinamate, meprobamate, methaqualome, cyclobenzaprene, cyclobenzaprine, tizanidine, baclofen, butalbital, zopiclone, atracurium, tubocurarine and phenobarbital antifungal, antiprotazoal and antiparasitic agents: including amphotericin, butoconazole nitrate, clotrimazole, econazole nitrate, fluconazole, flucytosine, griseofulvin, itraconazole, ketoconazole, miconazole, natamycin, nystatin, sulconazole nitrate, terconazole, tioconazole and undecenoic acid; benznidazole, clioquinol, decoquinate, diiodohydroxyquinoline, diloxanide furoate, dinitolmide, furzolidone, metronidazole, nimorazole, nitrofurazone, ornidazole, terbinafine, clotrimazole, chloroquine, mefloquine, itraconazole, pyrimethamine, praziquantel, quinacrine, mebendazole and tinidazoleantihypertensive and cardiac therapeutic agents: including candesartan, hydralazine, clonidine, triamterene, felodipine, gemfibrozil, fenofibrate, nifedical, prazosin, mecamylamine, doxazosin, dobutamine and cilexetil anti migraine agents: including dihydroergotamine mesylate, ergotamine tartrate, methysergide maleate, pizotifen maleate and sumatriptan succinate antimuscarinic agents: including atropine, benzhexol, biperiden, ethopropazine, hyoscyamine, mepenzolate bromide, oxybutynin, oxyphencylcimine and tropicamide antineoplastic agents (or immunosuppressants): including aminoglutethimide, amsacrine, azathioprine, busulphan, chlorambucil, cyclosporin, dacarbazine, estramustine, etoposide, lomustine, melphalan, mercaptopurine, methotrexate, mitomycin, mitotane, mitozantrone, procarbazine, tamoxifen citrate, testolactone, tacrolimus, mercaptopurine and sirolimus anti-Parkinsonian agents: including bromocriptine mesylate, levodopa, tolcapone, ropinirole, bromocriptine, hypoglycaemic agents such as sulfonylureas, biguanides, a-glucosidase inhibitors, thaiazolidinediones, cabergoline, carbidopa and lysuride maleate antithyroid agents: including carbimazole and propylthiouracil antiviral drugs: including amantadine, retinovir, cidofovir, acyclovir, famciclovir, ribavirin, amprenavir, indinavirm, rimantadine and efavirenz, penciclovir, ganciclovir, vidarabine, abacavir, adefovir, apmrenavir, delavirdine, didanosine, stavudine, zaicitabine, zidovudine, enfuvirtide and interferon cardiac inotropic agents: including amrinone, milrinone, digitoxin, digoxin, enoximone, lanatoside C and medigoxin hypo and hyper lipidemic agents: including fenofibrate, clofibrate, probucol, ezetimibe and torcetrapib antiinflammatory: including meoxicam, triamcinolone, cromolyn, nedocromil, hydroxychloroquine, montelukast, zileuton, zafirlukast and meloxicam antihistamine: including fexofenadine, chloral hydrate, hydroxyzine, promethazine, cetirazine, cimetidine, clyclizine, meclizine, dimenhydrinate, loratadine, nizatadine and promethazine antiulcer: including omeprazole, lansoprazole, pantoprazole and ranitidine diuretics: including hydrochlorothiazide, amiloride, acetazolamide, furosemide and torsemide opioids: including natural opiates which are alkaloids contained in the resin of the opium poppy such as morphine, codeine and thebaine; semi-synthetic opioids created from natural opiates such as hydromorphone, hydrocodone, oxycodone, oxymorphone, desomorphine, diacetylmorphine (heroin), nicomorphine, dipropanoylmorphine, benzylmorphine and ethylmorphine; fully synthetic opioids such as fentanyl, pethidine, methadone, tramadol and dextropropoxyphene; and, endogenous opioid peptides, produced naturally in the body, such as endorphins, enkephalins, dynorphins, and endomorphins; opioid analgesics including opioid receptor agonists, opioid receptor partial agonists, opioid antagonist or opioid receptor mixed agonist-antagonists; opioid receptor agonists including morphine, depomorphine, etorphine, heroin, hydromorphone, oxymorphone, levorphanol, methadone, levomethadyl, meperidine, fentanyl, sufentanyl, alfentanil, codeine, hydrocodone, oxycodone, and mixtures of the foregoing; opioid receptor antagonists including naloxone and naltrexone; opioid receptor mixed agonist-antagonist which has mixed opioid agonist/antagonist activities, or one that exhibits only partial agonist activity, including buprenorphine, nalbuphine, butorphanol, pentazocine, and mixtures of such compounds; opioids which exhibit partial agonist activity, including ethylketocyclazocine; opium alkaloids including phenanthrenes which are naturally occurring in opium such as codeine, morphine, thebaine and oripavine (the active metabolite of thebaine); synthetic derivatives such as diacetylmorphine (heroin), dihydrocodeine, hydrocodone, hydromorphone, nicomorphine, desmorphine, ethylmorphine, dipropanoylmorphine, oxycodone and oxymorphone; synthetic opioids including anilidopiperidines such as fentanyl, alphamethylfentanyl, alfentanil, sufentanil, remifentanil, carfentanyl and ohmefentanyl, phenylpiperidines such as pethidine (meperidine), ketobemidone, MPPP, allylprodine, prodine and PEPAP; diphenylpropylamine derivatives such as propoxyphene, dextropropoxyphene, dextromoramide, bezitramide, piritramide, methadone, dipipanone, levomethadyl acetate (LAAM), difenoxin, diphenoxylate and loperamide; benzomorphan derivatives such as dezocine, pentazocine and phenazocine; oripavine derivatives such as buprenorphine, dihydroetorphine and etorphine; morphinan derivatives such as butorphanol, nalbuphine, levorphanol and levomethorphan, and others such as lefetamine, meptazinol, tilidine, tramadol and tapentadol; opioid receptor antagonists including nalmefene, naloxone and naltrexone NSAIDs: including arylalkanoic acid sub-group of class which includes diclofenac, aceclofenac, acemetacin, alclofenac, bromfenac, etodolac, indometacin, indometacin farnesil, nabumetone, oxametacin, proglumetacin, sulindac and tolmetin; 2-arylpropionic acid (profens) sub-group of class which includes alminoprofen, benoxaprofen, carprofen, dexibuprofen, dexketoprofen, fenbufen, fenoprofen, flunoxaprofen, flurbiprofen, ibuprofen, ibuproxam, indoprofen, ketoprofen, ketorolac, loxoprofen, miroprofen, naproxen, oxaprozin, pirprofen, suprofen, tarenflurbil and tiaprofenic acid; and /V-arylanthranilic acid (fenamic acid) sub-group of class which includes flufenamic acid, meclofenamic acid, mefenamic acid and tolfenamic acid; tromethamine, celecoxib, nepafenac, aspirin, rofecoxib, naproxen, sulindac, piroxicam, pheylbutazone, tolmetin, indomethacin, acetominophen (paracetamol), tramadol and propoxyphene retinoids: including first generation retinoids such as retinol, retinal, tretinoin (retinoic acid, Retin-A), isotretinoin and alitretinoin; second generation retinoids such as etretinate and its metabolite acitretin; third generation retinoids such as tazarotene, bexarotene and adapalene hormones and steroids: including adrenocorticotrophic hormone (ACTH), antidiruetic hormone (vasopressin), atrial- nartreuretic factor (ANF), atrial-nartreuretic peptide (ANP), bedomethasone, cortisone, scopolamine, dopamine, epinephrine, catecholamines, cholecystokinin, clomiphene citrate, danazol, dexamethasone, diethylstilbestrol (DES), ethinyl estradiol, fludrocortison, finasteride, follicle stimulating hormone, gastrin, hydroxyprogesterone, growth hormone, insulin, leptin, luteinizing hormone, medroxyprogesterone acetate, mestranol, quinestrol, methyltestosterone, nandrolone, norethindrone, norethisterone, norgestrel, estradiol, conjugated oestrogens, oxandrolone, oxytocin, prednisone, progesterone, prolactin, protogalndins, somatostatin, stanozolol, stibestrol, thyroxine, prednisolone phosphate, triamcinolone, mifepristone acetonide, budesonide, levothyroxine, testosterone, testosterone cypionate, fluoxymesterone, flutamide, mometasone furoate, cyproterone, fluromethalone, goserelin, leuprolide, calcitonin, halobetasol, hydrocortisol and tibolone statins and derivatives: including atorvastatin, fluvastatin, lovastatin, nystatin, rosuvastatin, pravastatin, orlistat and simvastatin stimulants: including amphetamine, phentermine, tyramine, ephedrine, metaraminol, phenylephrine, dexamphetamine, dexfenfluramine, fenfluramine, nicotine, caffeine and mazindol vasocontrictors: including desmopressin vasodilitors: including carvedilol, terazosin, phentolamine and menthol antialzheimers: including levetiracetam, levitiracetam and donepezil ACE inhibitors: including benzapril, enalapril, ramipril, fosinopril sodium, lisinopril, minoxidil, isosorbide, rampril and quinapril beta adrenoreceptor antogonists: including atenolol, timolol, pindolol, propanolol hydrochloride, bisoprolol, esmolol, metoprolol succinate, metoprolol and metoprolol tartrate angiotensin II antagonists: including losartan platelet inhibitors: including abciximab, clopidrogel, tirofiban and aspirin alcohols and phenols: including tramadol, tramadol hydrochloride, allopurinol, calcitriol, cilostazol, soltalol, urasodiol bromperidol, droperidol, flupenthixol decanoate, albuterol, albuterol sulphate, carisoprodol, chlobetasol, ropinirol, labetalol, and methocarbamol ketones and esters: including amioderone, fluticasone, spironolactone, prednisone, triazodone, desoximetasone, methyl prednisdone, benzonatate nabumetone and buspirone antiemetics: including metoclopramide ocular treatments: including dorzolamide, brimonidine, olopatadine, cyclopentolate, pilocarpine and echothiophate anticoagulant and antithrombitic agents: including warfarin, enoxaparin and lepirudin treatments for gout: including probenecid and sulfinpyrazone COPD and asthma treatments: including ipratropium treatments for osteoporosis: including raloxifene, pamidronate and risedronate cosmetic peptides: including acetyl hexapeptide-3, acetyl hexapeptide-8, acetyl octapeptide and I-carnosine vaccines: including vaccines comprising toxoids (inactivated toxic compounds); proteins, protein subunits and polypeptides; polynucleotides such as DNA and RNA; conjugates; adjuvants such as saponins, virosomes, inorganic and organic adjuvants, for example zostavax nutraceutical and cosmeceutical actives: including coenzyme Q 0 (or ubiquinone), ubiquinol or resveratrol; a carotenoid such as [alpha], [beta], or [gamma]-carotene, lycopene, lutein, zeaxanthin and astaxanthin; a phytonutrient, such as lycopene, lutein and seaxanthin; an unsaturated fatty acid such as linoleic acid, conjugated linoleic acid, linolenic acid, omega-3 fatty acids including but not limited to docosahexaenoic acid (DHA) and eicosapentaeonic acid (EPA) and their glycerol-esters; fat-soluble vitamins including vitamin D (D2, D3 and their derivatives), vitamin E ([alpha], [beta], [gamma], [delta]-tocopherols, or a, [beta], [gamma], [delta]-tocotrienols), vitamin A (retinol, retinal, retinoic acid and derivatives), vitamin K (K-i, K2, K3 and their derivatives) capric/caprylic triglycerides, folic acid, iron, niacin, glyceryl linoleate, omega 6 fatty acids, vitamin F, selenium, cyanocobalamin, aloe vera, beta glucan, bisabolol, camellia thea (green tea) extract, capric/caprylic triglycerides, centella asiatica (gotu cola) extract, cetearyl olivate, chlorophyll, citrus sinensis (orange) oil, cocoyl proline, dicapryl ether, disodium lauriminodipropionate tocopheryl phosphates (vitamin E phosphates), glycerin, glyceryl oleate, glycyrrhiza glabra (licorice) root extract, hamamelis virgiana (witch hazel) extract, lactic acid, lecithin, lutein, macadamia integrifolia (macadamia) seed oil, matricaria chamomilla (chamomile) extract, Oenothera biennis (evening primrose) oil, olea europaea (olive) leaf extract, rice bran oil, persea gratissima (avocado) oil, polygonum multiflorum extract, pomegranate sterols, resveratrol, rosa eglanteria (rose hip) oil, santalum spicatum (sandalwood) oil, titanium dioxide, folic acid, glycerin, glyceryl linoleate (omega 6 fatty acids vitamin F), vitamin A palmitate, vitis vinifera (grapeseed) oil, halobetasol, adenosine, adenosine triphosphate, alpha hydroxy acid, allantoin, hyaluronic acid and derivatives, isolutrol, tranexamic acid, glycolic acid, arginine, ascorbyl glucosamine, ascorbyl palmitate, salicylic acid, carnosic acid, alpha lipoic acid, gamma linolenic acid (GLA), panthenol, retinyl propionate, retinyl pamitate, furfuryladenine, retinaldehyde, copper pepetides, idebenone, dimethylaminoethanol (DMAE), niacinamide, beta-glucan, palmitoyl pentapeptide-4, palmitoyl oligopeptide/ tetrapetide-7, ethocyn, ceramides, phenylalanine, glucuronolactone, L-carnitine, hydroxylapetite, palmitoyl tripetide-3, forskolin, zinc oxide, a-bisabolol, eugenol, silybin, soy isoflavones, aucubin, catalpol, pseudoguaianolide from Arnica chamissonis, rosmarinic acid, rosmanol, salicylates for example salicin, saligenin and salicyclic acid, taxasterol, a-lactucerol, isolactucerol, taraxacoside, ceremides, arbutin, gingerols, shagaols, hypercin, elastin, collagen and peptides thereof.

The active pharmaceutical ingredient may be incorporated in any form, *i.e.* as such, in salt form, as a hydrate or solvate, in crystalline form, amorphous form, liquid (solution) form or in a vehicle such as micelles, liposomes, microparticles and the like.

The active pharmaceutical ingredient may be incorporated into the composition of the invention before the curing step and/or it may be incorporated into a reservoir layer that is interposed between adhesive layer and backing layer

The relative amount of active pharmaceutical ingredient is not particularly limited. It is also possible to incorporate two or more active pharmaceutical ingredients in the same or separate layers.

A particular benefit of the present invention is that formulation can be cured a relatively low temperatures (e.g. room temperature) so that sensitive compounds may be included without the loss of efficacy that might be expected if curing was performed at elevated temperatures as used in the prior art.

### 7.3 Sterilization

The medical tape or patch of the present invention can also be sterilized if the final application requires it. Any known method of sterilization can be used, preferably one which does not affect adhesive strength by initiating a debonding reaction.

### 8. Examples

### Example 1: Polycondensation of dimer diacid and itaconic acid with the dimer diol

92.9 g of Pripol 1009, 21.3 g itaconic acid, 185.8 g of Pripol 2033 and 30mg 4-methoxyphenol were loaded in a 500 mL reactor equipped with a mechanical stirring system (using and "anchor" shaped stirrer), a nitrogen purge inlet, and Dean-Stark separator fitted with a condenser. Next, 30 mg of Ti(OⁿBu)₄ was added and the reaction mixture was stirred at 150 - 220 rpm. During the first part of the experiment, the set-up was continuously flushed with nitrogen (flow: 1 to 2 sL/min), the mixture was heated from room temperature to 180ºC and kept at this temperature for 15 hrs. Next, vacuum processing was applied (with typical pressure below 10 mbar), while the reaction temperature was increased to 160°C. After 2 hours under these conditions, the reaction temperature was increased to 180°C and kept under these conditions for 2 hours after which the heating temperature was set at 200ºC for an additional hour. Subsequently the pale yellow reaction mixture was allowed to cool under vacuum; once its temperature reached 130-140ºC, the reaction vessel was vented and discharged. Polycondensate A1 was thus obtained.

### Example 2: Chain extension reaction of an OH terminated polyester

10g of polycondensate A1 from Example 1 and 0.4g hexamethylene 1,6-diisocyanate were mixed together at room temperature. The obtained clear viscous paste was mechanically homogenized until disappearance of the air bubbles. Next this mixture was incubated at 50°C for 24 hrs. Polycondensate A2 was thus obtained.

**Table 1. Summary of the polymer properties (typical values).**

| **Polycondensate** | **Mn** | **Mw** | **PDI** |
|---|---|---|---|
| **A1** | 20782 | 37733 | 1.82 |
| **A2** | 25367 | 68165 | 2.69 |

### Example 3: Curing of polycondensate A2

10 g of polycondensate A2 from Example 2, 5.0g of Polysorb ID 37, 1.0g of 10 wt % stock solution of Irgacure 2959 in acetone were mixed together at room temperature. The obtained clear viscous paste was mechanically homogenized until disappearance of the air bubbles. Next the mixture was roll-coated manually at room temperature onto a PET liner and cured by exposure to UV-A and UV-B light (1.5 J/cm²) to obtain an adhesive composition as a film on the PET liner. A protective film was laminated onto the "free" side of the adhesive composition.

### Example 4: Peel force measurement

Bakelite substrates were used for these measurements. First, adhesive tape samples from Example 3 were cut in slices of 25 mm width and 60mm length. The tapes were secured using a 2 kg roller and a 30 min dwell time was allowed before removal. Next the tapes were peeled at 180 degrees at a rate of 300mm/min. The peel force values were measured using a Shimadzu Ag-X universal tensile testing machine. The peel force was found to be 0.80 N/25mm.

## Claims

1. A composition comprising:
a polycondensate (A); and optionally
an oil additive (B);
wherein the polycondensate (A) contains repeating units derived from:
a dicarboxylic acid (a);
a diol (b); and
an ethylenically unsaturated diacid (c);
wherein the weight average molecular weight of the polycondensate (A) is from 35,000 g/mol to 95,000 g/mol; and
if present, the amount of the oil additive (B) in the composition is from 0.01 to 1.5 parts by weight, preferably 0.2 to 1.2 parts by weight, based on 1 part by weight of the polycondensate (A).

2. The composition according to claim 1, further comprising a tackifier (C) in an amount from 0.001 to 0.3 parts by weight, preferably 0.01 to 0.05 parts by weight , based on 1 part by weight of the polycondensate (A).

3. The composition according to claim 1 or 2, further comprising an ethylenically unsaturated additive (D) in an amount from 0.01 to 0.8 parts by weight, preferably 0.05 to 0.3 parts by weight, based on 1 part by weight of the polycondensate (A).

4. The composition according to claim 1, 2 or 3, wherein the dicarboxylic acid (a) is a dicarboxylic acid represented by general formula (I):
R^{a}OOC-R¹-R²-R³-COOR^{b} (I)
wherein *R¹* is absent or selected from C₁₋₁₅ alkylene, C₁₁₋₁₅ alkenylene, and C₂₋₁₅ alkynylene, wherein each of these groups is optionally substituted; R² is selected from C₁₋₁₂ alkylene, C₁₁₋₁₂ alkenylene, and C₂₋₁₂ alkynylene, each of which may contain one or two 5-7-membered rings, which may optionally be condensed wherein each of these groups is optionally substituted; R³ is absent or selected from C₁₋₁₅ alkylene, C₁₁₋₁₅ alkenylene, and C₂₋₁₅ alkynylene wherein each of these groups is optionally substituted; each of R^{a} and R^{b} is independently selected from hydrogen, alkyl groups having 1 to 24 carbon atoms, and glyceryl

5. The composition according to any one of claims 1 to 4, wherein the diol (b) is a diol represented by general formula (II):
HO-R⁴-R⁵-R⁶-OH (II)
wherein R⁴ is absent or selected from C₁₋₁₅ alkylene, C₂₋₁₅ alkenylene, and C₂₋₁₅ alkynylene, wherein each of these groups is optionally substituted; R⁵ is selected from C₁₋₁₂ alkylene, C₂₋₁₂ alkenylene, and C₂₋₁₂ alkynylene, each of which may contain one or two 5-7-membered rings, which may optionally be condensed, wherein each of these groups is optionally substituted; R⁶ is absent or selected from C₁₋₁₅ alkylene, C₂₋₁₅ alkenylene, and C₂₋₁₅ alkynylene, wherein each of these groups is optionally substituted.

6. The composition according to any one of claims 1 to 5, wherein the dicarboxylic acid (a) is a dimer of fatty acids and/or the diol (b) is a dimer of fatty alcohols.

7. The composition according to any one of claims 1 to 6 wherein the ethylenically unsaturated diacid (c) is a compound represented by general formula (III):
R^{c}OOC-R⁷-COOR^{d} (III)
wherein R⁷ is selected from C₁₋₁₀ alkylene, C₂₋₁₀ alkenylene, and C₃₋₁₀ alkynylene, each of these groups being unsubstituted or substituted by 1-3 substituents, wherein said substituents are preferably selected from C₁₋₈ alkyl, C₁₋₈ alkenyl, C₂₋₈ alkynyl, C₁₋₆ alkoxy, and -R⁸-COOR⁹,
wherein R⁷ and/or at least one of its substituents is selected such that one or more carbon-carbon double bonds is/are present in the part of the molecule formed by R⁷ and its substituents, wherein such carbon-carbon double bond(s) may also be formed between a carbon atom of R⁷ and the carbon atom of the substituent, which is covalently bonded to said carbon atom of R⁷;
wherein R⁸ is C₁₋₃ alkylene, C₂₋₃ alkenylene or C₂₋₃ alkynylene and R⁹ is C₁₋₆ alkyl; and
wherein each of R^{c} and R^{d} is independently selected from hydrogen and alkyl groups having 1 to 6 carbon atoms.

8. The composition according to any one of claims 1 to 7 wherein the ethylenically unsaturated diacid (c) is selected from among itaconic acid and/or citraconic acid and/or mesaconic acid and/or aconitic acid, and/or their respective dialkyl esters, and/or any mixtures thereof.

9. The composition according to any one of claims 1 to 8, wherein the oil additive (B) is selected from sorbitan mono- or di-esters of fatty acids, polyethylene glycol diester, sesame oil, isosorbide diester, mixtures of isosorbide diesters, sorbitol mono- and diesters, isopropyl myristate, oleate esters or any mixture of these.

10. An adhesive composition comprising: a composition according to any one of claims 1 to 9 in which the polycondensate (A) has been at least partially cross-linked.

11. An adhesive composition according to claim 10, wherein the dicarboxylic acid (a) is represented by formula (I), the diol (b) is represented by formula (II), and the ethylenically unsaturated diacid (c) is represented by formula (III), and wherein the definitions for formulae (I)-(III) are as defined in the preceding claims.

12. The adhesive composition according to claim 10 or 11, wherein the cross-linked polycondensate (A) has a gel content value of from 40% to 95%, preferably from 60% to 80%.

13. A substrate, preferably a medical tape or a medical patch, comprising the composition according to any one of claims 1 to 9 or the adhesive composition according to any one of claims 10 to 12.

14. A method for producing the adhesive composition according to any one of claims 10 to 12, comprising the steps of:
(i) preparing and/or providing a composition according to any one of claims 1 to 9, wherein the composition optionally comprises a cross-linking agent; and
(ii) subjecting the composition of step (i) to conditions such as to cause the polycondensate (A) to at least partially cross-link.

15. A method for producing a medical tape or a medical patch with an adhesive coating, comprising the steps of:
(i) coating a composition according to any one of claims 1 to 9 onto a substrate, wherein the composition optionally comprises a cross-linking agent; and
(ii) subjecting the composition of step (i) to conditions such as to cause the polycondensate (A) to at least partially cross-link.

16. The method according to claim 15, wherein
the coating in step (i) is onto a liner, and after step (ii) the side of the adhesive coating opposite the liner is attached to a backing, support or protective film.

17. The method according to any one of claims 14 to 16 wherein the conditions that cause cross-linking include irradiation by UV light, electron beam, gamma-ray and/or X-ray.
